# EUROPÄISCHE PATENTSCHRIFT

(11) **EP 1 325 750 B1**
(45) Veröffentlichungstag und Bekanntmachung des Hinweises auf die Patenterteilung: **12.05.2004**
(21) Anmeldenummer: 02000367.9
(22) Anmeldetag: 05.01.2002
(51) Int. Cl.: A61K 35/78, A61P 17/14

(54) **Herstellung von Medikamenten (keine Lotion) aus Olivenöl als Haarwuchsmittel**
Preparation of medicines (not lotions) based on olive oil for use as hair growth aid
Préparation de médicaments (pas de lotion) à base d'huile d'olive comme produit capillaire

(43) Veröffentlichungstag der Anmeldung: 09.07.2003
(73) Patentinhaber: Schneider, Henning, 38678 Clausthal-Zellerfeld (DE)
(72) Erfinder: Schneider, Henning, 38678 Clausthal-Zellerfeld (DE)

(56) Entgegenhaltungen:
- DE-U- 20 118 366
- DATABASE BIOSIS [Online] BIOSCIENCES INFORMATION SERVICE, PHILADELPHIA, PA, US; 1991 WATANABE S ET AL: "HAIR GROWTH ON NUDE MICE DUE TO CYCLOSPORIN A" Database accession no. PREV199293129504 XP002203130 & JOURNAL OF DERMATOLOGY (TOKYO), Bd. 18, Nr. 12, 1991, Seiten 714-719, ISSN: 0385-2407
- DATABASE BIOSIS [Online] BIOSCIENCES INFORMATION SERVICE, PHILADELPHIA, PA, US; 1991 SALERNO J W ET AL: "THE USE OF SESAME OIL AND OTHER VEGETABLE OILS IN THE INHIBITION OF HUMAN COLON CANCER GROWTH IN-VITRO" Database accession no. PREV199192007202 XP002203131 & ANTICANCER RESEARCH, Bd. 11, Nr. 1, 1991, Seiten 209-216, ISSN: 0250-7005

## Beschreibung

Im Stand der Technik wird Olivenöl bisher nur als Zusatzmittel in Haar-Lotionen verwendet. (siehe DE 197 11 950 A1, DE 2 145 204 A, DE 2 107 641 A, FR 2 642 966 A1, GB 2 216 415 A, GB 1237 656, CA 2 132 170 A1, Derwent-Ref.: 1998-462748, Chem. Abstr.: 129:320935, Derwent-Ref.: 1993-189691.

Die Anwendung von Haar-Lotionen ist grundsätzlich durch die damit verbundenen Einwirkzeiten zeitaufwendig. Es ist deshalb wesentlich einfacher, wenn das Problem des Haarausfalles durch die Einnahme von Medikamenten von innen her behandelt wird.

Durch die 2 mal tägliche Einnahme eines Teelöffels mit reinem Olivenöl wird der Haarausfall gestoppt und es kommt auch zur Neubildung von Haaren.

Als Haarwuchsmittel stelle ich mir die Herstellung von unterschiedlichen Medikamenten vor, deren einziger Bestandteil aus Olivenöl besteht. Diese Form von Medikamenten sollen unter Schutz gestellt werden (siehe Ansprüche). Von Watanabe S. et al. (1991, Database Biosis Online; "Hair Growth on Nude Mice due to Cyclosporin A") wird die orale Verabreichung von Cyclosporin A gelöst in Olivenöl beschrieben, was zu Haarwachrotum bei nackten Mäusen führte. Im Unterschied dazu wird von mit reines Olivenöl als einziger Bestandteil zur Herstellung von Medikamenten gegen Haarausfall und als Haarwuchsmittel oral oder in Form von Zäpfchen verwendet.

Da Olivenöl ein rein pflanzliches Produkt ist und sowieso als Nahrungsmittel vermarktet wird, sollte es keine Schwierigkeiten bei der Zulassung als Medikament geben. Mir sind keine negativen Erscheinungen bekannt, die gegen die Einnahme von Olivenöl sprechen.

### Beispiele für erzielte Erfolge:

Seit knapp 18 Monaten nehme ich 2 mal täglich einen Teelöffel reines Olivenöl zu mir.

Ich werde in ein paar Tagen 48 Jahre alt und habe mittelblonde und auch sehr dünne Haare. Es ist wissenschaftlich nachgewiesen, dass die für den Haarwuchs verantwortlichen Gene bei blonden Menschen schwächer sind als bei dunkelhaarigen. Trotz dieser ungünstigen Voraussetzungen bei mir, haben sich deutliche positive Erfolge gezeigt:
- der Haarausfall ist gestoppt:(es befinden sich nach der Haarwäsche keine kompakten Haarbüschel mehr auf dem Abflußsieb der Dusche).
- Vergleichmäßigung des einst sehr unregelmäßigen Haarwuchses: (In Abständen von ca. 8 Wochen laße ich mir die Haare schneiden. Bevor ich mit der Einnahme von Olivenöl begonnen hatte, sind meine Haare sehr ungleichmäßig nachgewachsen, d.h. ich hatte einige überlange Haarsträhnen und andere Haare, die längenmäßig im Wachstum zurückgeblieben waren. Seit mehreren Monaten wachsen meine Haare sehr gleichmäßig nach).
- deutliche Haar-Verdichtung im Tonsurbereich (an dieser Stelle sind mir zuletzt die Haare lichter geworden. Mittlerweile hat sich hier die volle Haarpracht wieder eingestellt).
- Neubildung von Haaren auch auf den Schläfen (mir ist kein Haarwuchsmittel bekannt, dass eine Neubildung auf den Schläfen verspricht. Ich habe erreicht, dass sich bei mir ein gleichmäßiger, allerdings nicht allzu dichter Flaum gebildet hat. Es sind auch einige neue Haare dabei, die annähernd die normale Länge erreicht haben).
- Verdichtung der vorderen Haare oberhalb der Schläfen (in diesem Bereich sind mir meiner Meinung nach zuerst die Haare ausgegangen. Auch dieser Bereich ist schon etwas voller geworden und verdichtet sich weiterhin).
- Allgemein kann ich feststellen, dass sich die Haare in umgekehrter Reihenfolge wieder zurückbilden, wie Sie mir einst ausgefallen sind (d.h. die Haare, die zuletzt ausgefallen sind, bilden sich zuerst zurück, während sich die Haare, die zuerst ausgefallen sind, sich zumindest teilweise als letztes wieder zurückbilden).

Ich möchte noch darauf hinweisen, dass sich die positiven Auswirkungen des Olivenöls bei dunkelhaarigen und auch bei jüngeren Personen sicherlich noch deutlicher zeigen würden.

Zudem versichere ich Ihnen, dass die hier von mir gemachten Angaben sachlich richtig sind, auch wenn es unglaubwürdig klingen mag. Es gibt einige Personen in meinem Bekanntenkreis, die meine gemachten Aussagen bestätigen können. Zu diesem Personenkreis gehören auch 2 Frisöre.
Einige der geschilderten Erfolge kann ich durch unmanipulierte Fotos belegen.

## Patentansprüche

1. Verwendung von reinem Olivenöl als einziger Bestandteil zur Herstellung von Medikamenten gegen Haarausfall wird als Haarwuchsmittel, wobei die Medikamente oral oder als Zäpfchen verabreicht werden.

2. Verwendung nach Anspruch 1, wobei die medikamente
- als Trinkampulle,
- als Kapsel,
- als Tropfen oder
- als Zäpfchen
vorliegen.

## Claims

1. Application of pure olive oil as the only component for the preparation of medicines against loss of hair and for the use as hair-restorer. Medicines for oral taking or as suppositories.

2. Application according to item 1.:
as ampoule,
as capsule,
as drops or
as suppository

## Revendications

1. Utilisation d'huile d'olive pure comme seul composant pour la préparation de médicaments contre la chute des cheveux et comme produit favorisant la pousse des cheveux. Ces médicaments peuvent être cependant administrés soit par voie orale, soit sous forme de suppositoires.

2. Utilisation d'après la description no 1. mais aussi
- comme ampoule buvable,
- comme gélule (capsule),
- comme gouttes,
- ou comme suppositoires
